# EUROPEAN PATENT SPECIFICATION

(11) **EP 4 188 213 B1**
(45) Date of publication and mention of the grant of the patent: **12.03.2025**
(21) Application number: 21769496.7
(22) Date of filing: 30.07.2021
(51) Int. Cl.: A61B 5/07, A61B 5/103, A61B 5/11, A61B 5/00

(54) **A SYSTEM FOR MONITORING A PATIENT**
SYSTEM ZUR ÜBERWACHUNG EINES PATIENTEN
SYSTÈME DE SURVEILLANCE D'UN PATIENT

(30) Priority: 31.07.2020 GB 202011995; 21.12.2020 GB 202020209; 20.04.2021 GB 202105609
(43) Date of publication of application: 07.06.2023
(73) Proprietor: Real Implants Ltd, London Greater London N12 0DR (GB)
(72) Inventor: ZAMAN, Tariq, London Greater London N12 0DR (GB)
(74) Representative: IK-IP LTD
(86) International application number: PCT/GB2021/051987
(87) International publication number: WO 2022/023775

(56) References cited:
- WO-A1-2016/065205
- WO-A1-2022/018652
- US-A1- 2008 300 597

## Description

### FIELD OF THE INVENTION

The present application relates to monitoring and treatment of musculoskeletal injuries. In particular, embodiments relate to systems and methods for monitoring strain in a musculoskeletal element.

### BACKGROUND OF THE INVENTION

Musculoskeletal injuries are a common condition around the world, and the incidence of such cases, particularly bone fractures, is likely to only increase with ageing populations. The treatment of injuries presents large financial costs to already-strained healthcare systems, as well as considerable disruption to the lives of patients and their carers and families.

Some bone fractures are treated using implants to fix sections of bone while the bone heals. Similarly, damaged soft tissues such as tendons and ligaments are often treated surgically using tissue grafts to reconstruct the damaged tissue. In addition, joints may be replaced, such as hips and knees. In the months that follow surgery, patients must pay multiple visits to potentially distant clinics in order to monitor the progress of their healing. Such visits often include repeated scans such as x-ray and ultrasound, which increase costs for the healthcare system and, for bone fractures, radiological exposure for patients. These visits often require patients and people around them to take time off work, adding to the national cost of fracture treatment.

There have been proposals for fracture fixation devices to be used with sensors to monitor the load borne by the fixation device. Such sensors should in principle allow fracture healing to be monitored, since the bone will take more load and the fixation device less load as the fracture heals. However, proposals in the field of surgery have not developed into satisfactory real-world solutions due to a number of practical issues which have not been fully addressed. For example, the inventor, as an orthopaedic surgeon, is acutely aware of the importance of periodic assessment in musculoskeletal treatment, yet current arrangements require patients to visit clinics regularly for monitoring and assessment. One particular issue is that in experimental tests a sensor is applied to a fracture in a known location by a researcher and the experiment is bespoke and has other experimental attributes (for example cumbersome trailing wires) which mean that is not readily reproducible in real world applications.

US 2008/300597 discloses an instrumented orthopaedic implant, such as an intramedullary (IM) nail. The implant has the capacity to provide an accurate measurement of the applied mechanical load across the implant. The implant includes sensors and associated electronic components located in recesses on the outer surface of the implant. The implant houses the sensing apparatus, the interface circuitry, the data transmitter, and the power receiver. The hermetically sealed housing is adapted for implantation in the body of a patient. The implant is used with a controller which communicates with it by telemetry, and there is an acting unit connected to the electronic components which is adapted to carry out a function based upon a condition detected by the sensors. WO 2016/065205 discloses an implant body, an actuator coupled to the implant body, a sensor configured to detect a parameter indicative of a biological condition, a transceiver, and a controller. The transceiver is configured to transmit data associated with the parameter to an external remote control and receive instructions from the external remote control. The controller is configured to move the actuator in response to the instructions from the external remote control, wherein the actuator adjusts the implant body,

### SUMMARY OF THE INVENTION

Pursuant to the invention it has been appreciated that there is scope for a better long-term monitoring and measurement system for application to real world mobile patients, not just laboratory situations.

Aspects of the invention are set out in the independent claim and preferable features are set out in the dependent claims.

A first aspect is set out in Claim 1.

The system can allow strain of musculoskeletal elements (which may include bones, ligaments, tendons or even whole joint replacements), and therefore healing of fractures and ruptures, to be periodically monitored safely and reliably over an extended period of time without the need for scans. Thus, the amount of radiological exposure the patient receives during treatment can be reduced. Thus, measurements can be safely and conveniently taken at least once a week: this can allow healing to be determined sooner than in existing clinical practice, which can facilitate faster treatment of such conditions. The user device being capable of communicating wirelessly with the sensing device allows a series of measurements to be taken in real-time without the need for wired connections, which can improve the ease of use of the system and with implanted structures can reduce the risk of infection,

particularly over longer time periods such as the 12 to 16 weeks typically required for many bone fractures to heal.

Preferably the distributed strain sensor comprises an optical sensor arranged to be physically coupled to the fixation device (or bone reinforcement structure) to be implanted in the patient secured to a fractured bone or other injured element (for example a torn muscle), the optical sensor comprising an optical fibre, a light source and a light detector. A measure of strain is determined from the light detector based on light transmitted through the optical fibre.

Optical sensors can be less intrinsically susceptible to infection and attack in the body but there is not such a direct correlation between the condition of the material and the strain. However, the present system can provide a reliable method for use in monitoring a live patient for an extended period.

More generally an optical fibre can if appropriately configured with a sensor be used to sense deformation throughout its length by a change in optical properties from bending, stretching or crushing and can relatively easily be rendered inert to a body environment thus lending itself to the sensing task well. However alternative sensors which achieve the same function can be used. Resistance based strain sensors are generally easy to implement in a circuit and have relatively low intrinsic power requirements so in principle might be thought to be better suited to a medical application where monitoring over a period of time is required. However, finding practical arrangements which reliably monitor strain while resisting the internal body environment and without presenting risk of infection is problematic. Nonetheless we have found that with suitable encapsulation strain sensors may be used.

Another issue is that resistance strain sensors are generally better at measuring localised strain and if a sensor is pre-manufactured the point of fracture is not known at the time of manufacture. If a resistance-based sensor is used it is preferable to have a plurality of sensors distributed along the implantable device and processor may be arranged to derive a signal for a patient indicative of movement in the region of the fracture from the plurality of signals. For example, the processor may apply a weighting function to signals from multiple sensors to emphasise signals from the sensor closes to the injury or fracture, or may simply select the sensor closest. The selection may be programmed e.g. around the time of implantation based on visualisation of where the fracture or injury is in relation to the sensor or the processor may determine or learn the sensor or combination of sensors giving the best signal, for example in a training or initial stage then use this combination for future measurements for consistency.

The alerting device being arranged to provide an audible and/or visual alert to a user to adopt a predetermined pose allows the musculoskeletal element to be loaded under controlled conditions and does not require the assistance of a medical or veterinary professional for a measurement to be obtained. Thus, strain can be determined reliably in extra-clinical environments.

By arranging the processor to control the power supply arrangement to power the optical sensor selectively, and to receive a reading and transmit a signal based on the reading, strain can be monitored as required in real time, for example during physiotherapy or controlled loading of the musculoskeletal element, but so as to power the optical sensor only when a measurement is required. This can allow the power supply arrangement either to be a passive device, or can allow the power of an implanted battery to be conserved in order to provide sufficient power to monitor over an extended period of time.

The term "light detector" as used herein refers to a component capable of converting incident light to corresponding electrical signals, the component including but not limited to a photodiode, photoresistor, phototransistor and/or photomultiplier tube.

The term "patient" as used herein refers to an animal such as a human or any vertebrate animal having a fractured bone being or to be treated. The term "user" as used herein refers to any person, entity or other system operating the user device. As such, a human may be both the patient and the user if the human is using the user device in the system for treating a fracture to one of their bones. Alternatively, the user may be a clinician or technician, either positioned with the patient or remotely.

The term "musculoskeletal element" as used herein refers to a tissue, structure or organ which is part of the musculoskeletal system. Musculoskeletal elements include bones or any tissue, structure or organ connecting to, controlling or supporting bones. Musculoskeletal elements include but are not limited to bones, muscles, tendons, ligaments, cartilage and joints.

The musculoskeletal element may be a damaged internal tendon or ligament. In the case of a tendon or ligament, the strain sensor may measure only tension in which case a single strain sensor may suffice to measure elongation of the support. However, the musculoskeletal element may be a fractured internal bone. In such a case the strain sensing arrangement may be arranged to measure bending strain and in such a case it is preferred to have multiple discrete strain sensing elements or an optical fibre. The sensing device may be implantable and encapsulated in a biocompatible housing or coating. The biocompatible housing or coating can protect the sensing device from a harsh biological environment within the patient and can protect internal tissue of the patient from becoming damaged or infected. The reinforcement structure may be a bone reinforcement structure. The bone reinforcement structure may be fully implanted in the patient. The short-range wireless transceiver may be arranged to communicate digital data through the skin of the patient. This can allow measurements to be obtained from within the body of the patient without the need for wired connections. The musculoskeletal element may be a fractured pelvis or a hip joint. The system may be used to monitor strain over a period of at least 24 weeks or at least 24 times.

Where the system is applied to joint replacement, strain of a component may be measured but other parameters such as torsion or shear stress (which may show themselves as strain of a component of the replacement joint) may be measured more directly by a dedicated sensor.

The system will indicate to a patient to adopt a predetermined pose (which may include performing a particular exercise or activity which cycles through multiple poses). However in some cases the system may monitor activity continuously or periodically or for example triggered by movement. For example if an externally worn sensor detects that a patient is walking or running this may trigger a measurement. An internal movement sensor may trigger taking and storing of a measurement in such a case for later reading.

The indication to adopt a predetermined pose may comprise an indication to perform an activity which implicitly includes a sequence of poses, for example walking or a particular exercise from which the processor obtains a representative measurement as parameters vary.

The processor or the remote server may be configured to detect a potential malfunction of the device or a problem condition (for example a reinforcement coming lose) when insufficient deformation is detected or a pattern that is implausible (for example suggesting unusually fast healing) is detected.

The processor or the remote server may be configured to detect a problem with the patient recovery when excessive deformation is detected or a pattern that suggests unusually slow healing is detected.

The processor or the remote server may include a machine learning module trained to detect patterns associated with be configured to detect a potential malfunction of the device or a problem condition (for example a reinforcement coming lose) when insufficient deformation is detected or a pattern that is implausible (for example suggesting unusually fast healing) is detected.

The processor may maintain the power supply arrangement in a low power state in which substantially no power is provided to the optical sensor. By maintaining the power supply arrangement in a low power state when not transmitting reading signals, damage to tissues surrounding the sensor caused by heat or other electromagnetic signals can be minimised or eliminated, and in a case where the power supply arrangement is an implanted power source such as a battery, the available power in the power supply arrangement can be conserved allowing the system to be used for extended periods of time. The power supply arrangement may be arranged to provide power to the optical sensor in response to receiving the signal to trigger powering the optical sensor. This can allow a more helpful series of measures to be obtained from a fully implanted sensor over an extended period, thereby reducing the risk of infection over wired connections.

The user device can be provided by an application running on a mobile device. The mobile device may be one of a smartphone, a tablet, a computer or a smart watch. Mobile devices such as these are commonly available, so providing the user device on an application can increase the accessibility of the system, further reducing the need for specialist equipment. The first communication interface may be one of a Bluetooth^{®} Low Energy (BLE) interface, a near-field communication (NFC) interface, or a Zigbee^{®} interface. The first protocol may be one of Bluetooth^{®} Low Energy (BLE), near-field communication (NFC) or Zigbee^{®}. This can allow the user device to communicate with the short-range wireless transceiver using a low-energy communication protocol, meaning the short-range wireless transceiver requires less energy to operate. The second communication interface may be one of a Wi-Fi^{®} interface, a 3G interface, a 4G interface, a 5G interface or an ethernet interface.

The user device may be configured to determine whether the patient has adopted the predetermined pose using one or more sensors and/or one or more indications from the user that the patient has adopted the predetermined pose. The first communication interface may be configured to send the signal to trigger the processor to power the optical sensor once the patient has adopted the predetermined pose. This can ensure that the fractured bone is correctly loaded under controlled or predetermined conditions before powering the optical sensor, allowing strain measurements to be obtained more reliably.

The user device may be configured to pause for a predetermined length of time after providing the alert before sending the signal to trigger the processor to power the optical sensor. This can give the patient time to adopt the predetermined pose. When pausing, the alerting device may be configured to provide a countdown to the user to indicate that the user device is about to send the signal to trigger the processor to power the optical sensor. At the end of the predetermined period, it is presumed that the patient is in position in the predetermined pose so that the sensor can be powered and a reading taken.

The user device may have an input for receiving a signal indicating that the patient is in the predetermined pose. The signal may be generated by the patient themselves or by an operator of the device for example by pressing a button. The first communication interface may be configured to send the signal to trigger powering the optical sensor upon the user device receiving the signal indicating that the patient is in the predetermined pose.

The user device may be configured to send the signal to trigger the processor to power the optical sensor after providing the alert.

The processor may be configured to receive a plurality of readings over a short time interval, wherein the reading signal is based on the plurality of readings. The plurality of readings may comprise at least two, preferably at least three readings, and the short time interval may comprise a window of 5 seconds or less. The processor, the user device and/or the server may be configured to determine that the measurement data is stable if each of the plurality of readings is within a threshold, preferably with a variation of less than 5%, preferably of less than 1%. The user device may be configured to indicate a fault or that a reading should be retaken if any of the plurality of readings is outside the threshold. This can allow more reliable strain measurements to be obtained by taking readings spanning a time period during which the patient is likely to have adopted the predetermined pose. The strain in the structure in the predetermined pose can therefore be determined from the plurality of readings, for example by using the reading indicative of a maximum strain. By determining whether the data is stable allows erroneous readings to be identified and discarded. The user device may be arranged to send a signal to the server to indicate an apparatus fault if successive readings are outside a threshold consistent with estimated likely healing.

The power supply arrangement may be arranged to receive power from an external power source, preferably inductively using an energetic signal. Receiving power externally allows the sensing device to comprise entirely passive components, meaning the power supply arrangement may not include a power source such as a battery, which can reduce the risk of infection and/or or harm to the patient resulting from toxic materials or excessive dissipated energy. Being passive can also make the encapsulation and implantation of the sensing device simpler. The power supply arrangement may comprise a capacitor configured to charge inductively using the energetic signal. The capacitor may be a supercapacitor, preferably having an electrical conductivity of at least 1500 Siemens per metre. The supercapacitor may comprise electrodes made from graphene. The energetic signal may have a power of around 500 µW or less and may be transmitted for a duration of around 2 seconds or less, preferably around 1 second. Using a capacitor can allow a relatively low-power energetic signal to be used for a length of time to build up sufficient energy in the capacitor in order to power a higher-power light source such as a laser diode for a shorter length of time. This can allow lower-power technology to be used to transmit the energetic signal and may also prevent damage through excessively heating the patient's bodily tissue. The energetic signal may be a radio-frequency signal.

The user device may further comprise apparatus for transmitting the energetic signal to power the sensing device inductively. The apparatus may comprise a radio-frequency transceiver provided on the user device, the transmitter arranged to transmit the energetic signal through the skin of the patient. This can allow measurements to be taken from the sensor without requiring specialist medical equipment and can allow the strain to be monitored in situations where wired device connections or bulky equipment are not available or practical. The power supply arrangement may be arranged to receive power inductively from the apparatus. This can allow both communication with the controller and inductive power transmission to be performed using a single device, such as a smartphone.

Alternatively, the system may further comprise a power device for transmitting the energetic signal to power the sensing device inductively. The power device may comprise a radio-frequency transceiver arranged to transmit the energetic signal through the skin of the patient. Power may be supplied from an external arrangement with an inductive power coupling, such as an armband or leg band or other extended coil or antenna arrangement positioned near the implanted sensor. By having an extended external coil or antenna, sufficient coupling may be achieved to transmit sufficient power to a very compact implanted sensor which does not have a large antenna or coil. This may be achieved at relatively low frequencies (of the order of a few tens or hundreds of kilohertz) which are considered not to interact with the patient tissue at the power levels involved.

The power supply arrangement may be arranged to receive power inductively from the power device. This can allow various types of mobile devices to be used for communicating with the sensing device without requiring such mobile devices to be capable of also transmitting energetic signals, for example using a radio-frequency transceiver. The short-range wireless transceiver may be arranged to communicate with the user device directly. The user device may be arranged to send an activation signal to the power device to cause the power device to transmit the energetic signal. This can allow the power device to be controlled by the user from the user device.

Alternatively or additionally, the processor may be arranged to communicate with the user device via the power device. The power device may further comprise a second shortrange wireless transceiver. The first communication interface may be arranged to send the trigger signal to the power device. The power device may be arranged, in response to receiving the trigger signal, to transmit the energetic signal to the sensing device and to send the trigger signal to the processor. The processor may be configured to transmit the reading signal to the external user device via the power device. The power device may be arranged to transmit the reading signal to the user device. This can allow the user device to communicate directly only with the power device and remove the need for the user device to transmit signals through the skin and other bodily tissues of the patient. Thus, the power device can be used to amplify signals received from the sensing device or the user device, and/or, for a given signal, can use different modalities when communicating with the user device and the sensing device. This can allow the communication modality to be selected for the transmission medium, namely bodily tissue or air, such that transmission losses are reduced or minimised. The second short-range wireless transceiver may be configured to communicate using at least one of radio-frequency signals, a near-field communication (NFC) protocol, a Bluetooth^{®} protocol, and a Bluetooth^{®} Low Energy (BLE) protocol.

The power supply arrangement may comprise a battery. This can allow the power supply arrangement upon implantation into the patient to contain the power required to periodically power the light source over an extended period. Therefore, the sensing device only needs to receive information signals through the skin of the patient rather than power as well, thus allowing only low-power signals to be transmitted through the patient. The processor may be configured to power up the optical sensor at predetermined intervals and create a log of readings in a memory. The processor may be configured to transmit the log of readings upon receiving a request from the user device. By creating a log of readings and storing it in a memory, a series of measurements can be made automatically and/or at predetermined times, improving for the user the ease of monitoring strain. For example, the processor may obtain readings during the night weekly and provide these to the user device when the user, a clinician or a technician requires to access the log.

The power supply arrangement may be configured to provide power to the optical sensor for a predetermined length of time, preferably no more than 10 ms, more preferably around 1 ms. This can ensure that the optical sensor automatically returns to a lower power state after transmitting the reading signal. The power supply arrangement may be configured to cease providing power to the optical sensor in response to receiving a stop signal from the user device. This can allow the sensor to wait for confirmation that no more measurements are required before returning to a lower power state.

The patient may be an animal, preferably a human. The structure may be a bone fixation plate for treating the fractured bone. This can allow patients requiring fixation of fractured bones to monitor the healing of their fracture by measuring the strain of the fixation plate, which can allow the load borne by the healing bone to be inferred.

The audible and/or visual alert to the user may comprise at least one of a textual and/or audio instruction, a graphic, an animation, a light, or a sound. Thus, the device can be used to clearly depict and/or describe to the user the pose required in order to produce the correct controlled loading conditions of the bone without requiring a medical professional.

The measurement data may be communicated to the remote server in an encrypted format. The remote server may be configured to store the measurement data in a database in the encrypted format. The measurement data may be accessible from the database. The server may be arranged to provide access to the measurement data only when provided with a security corresponding to the measurement data and/or the patient. This can allow patient data to be transmitted to, stored at and accessed from remote locations securely, reducing the risk of personal patient data being stolen or otherwise compromised.

The server may be configured to determine a healing condition of the fracture based on the measurement data. In determining the healing condition, the server may compare the measurement data with one or more thresholds and/or one or more reference signals. The one or more thresholds may include a value one standard deviation away from a mean value of a sample of reference data. The server may comprise a learning engine trained using a training dataset, the learning engine configured to receive input data including the measurement data and to provide as output the healing condition, preferably a classification or a value indicative of treatment progress. By analysing measurement data at the server, greater computing power may be employed than that available to the user device and databases storing data such as medical history may be accessed more securely. The input data may further include at least one of data relating to physician availability, the patient's medical history, and population healing data. The learning engine may make a treatment decision automatically based on at least a portion of the input data and/or the healing condition. The learning engine may be configured to determine whether a treatment is advancing normally. This may allow the patient to progress to another stage of treatment without requiring a visit to a clinic, or the healing condition may be used to decide that the patient should visit the clinic for further assessment or treatment. Thus, the need for healthcare resources such as professional consultation and scanning is removed or reduced from this monitoring stage of treatment, increasing availability of such resources for patients whose fracture treatment is not advancing normally and/or reducing costs for healthcare systems. Using a learning engine can allow healing conditions and/or treatment decisions to be determined with greater throughput than with human professional assessment, meaning the healing conditions and/or treatment decisions for individual patients can be determined faster, thus reducing the risk of conditions such as fracture implant failure by allowing, for example, impending non-unions to be detected sooner with earlier clinical referral. Alternatively or additionally, a clinician may access the measurement data from the server and analyse the measurement data to determine a healing condition remotely. A clinician may access the measurement data using the security key.

The term "healing condition" as used herein refers to a status or degree of healing of the fracture to the internal bone. A healing condition may be quantitative including but not limited to a mechanical property of the bone, a load borne by the bone or a value indicative of calcification. A healing condition may be qualitative, including but not limited to a medical grading score indicative of healing progress or a category indicative of healing such as callus formation. In one embodiment, the "healing condition" may be a numerical, textual or graphical indication based on an estimation of how far through the healing process the bone of the patient has progressed. For example, measurement data obtained by the sensor may be converted into an indication of the progress of the healing (the healing condition) as the bone state changed from fractured to healed.

The server may be configured to transmit the healing condition to the user device. This may include assessing soft tissue repair, detecting possible infection (for example based on temperature) or inferring possible loosening of the reinforcement device (for example from unexpectedly or anomalously reduced strain measurement). The alerting device may be arranged, in response to receiving the healing condition, to update the alert such that it indicates that the patient should adopt a second pose. The first communication interface may be configured to send the signal to trigger the processor to power the optical sensor once the patient has adopted the second pose. The user device may be configured to determine whether the patient has adopted the second pose using one or more sensors and/or one or more indications from the user that the patient has adopted the predetermined pose. This can ensure that the fractured bone is correctly loaded under a second set of controlled or predetermined conditions before powering the optical sensor. This can allow different poses to be adopted depending on the stage of healing. For example, **if** the healing condition indicates an increased callus formation then a patient may be instructed to adopt poses inducing higher loads on the bone and/or fixation plate. This can allow more and/or different information to be obtained from the reading signals as the fracture heals and may facilitate the acceleration of a treatment process.

The server may be configured to communicate with the device using a wide area network (WAN), preferably the Internet. The server may be configured to communicate with the user device using a combination of one or more wide area networks (WAN) and one or more local area networks (LAN), preferably one or more wireless local area networks (WLAN).

The light source may be a laser such as a laser diode. The light source may produce monochromatic light. The light source may produce infrared, visible and/or ultraviolet light. The light source may require a power of less than 500 mW to produce light.

At least a portion of the optical fibre may comprise a Bragg grating. This can allow the light detector to be configured to detect the light from the light source reflected by the Bragg grating. Thus, the light detector can be positioned at a same end of the optical fibre as the light source, reducing or minimising the amount of wiring required in the optical sensor, allowing a more compact layout of the optical sensor.

The optical sensor may comprise a photonic integrated circuit. One or both of the processor and transceiver may be integrated on a single photonic integrated circuit with the optical sensing arrangement. Preferably the entire sensing arrangement (potentially excluding antennae) may be integrated on a single application specific integrated circuit. Such circuits may be sensitive to bending of the circuit itself - this property may be used additionally or alternatively to strain in the fibre to measure strain.

The sensing device may be physically coupled to an outer surface of the structure. The sensing device may be physically coupled to a surface of an internal cavity of the structure. The sensing device may be physically coupled to the structure using a biocompatible adhesive, preferably a silicone. Other biocompatible adhesives may be used, including polymethyl methacrylate. The biocompatible adhesive may provide the biocompatible housing or coating. The biocompatible housing or coating may be integrally moulded with the structure. This can ensure that the sensing device is protected from the harsh biological environment while being securely fixed to the structure, and that the surrounding tissue is protected from any reactive or toxic materials in the sensing device. Silicones provide particularly strong bonding between metal and glass, which may allow secure fixation of the optical fibre to a metal fixation device.

The optical fibre may have a diameter of no more than 5 mm, preferably less than 1 mm, more preferably around 0.5 mm or less. The small size of the optical fibre may allow the sensing device to be attached to a wide range of bone reinforcement structures without causing obstruction to pins or other mechanical features of the structures. For example, the sensor may be fixed to a narrow side face of a fixation plate. The power supply arrangement, the short-range wireless transceiver, the processor and circuitry of the optical sensor may be disposed in a different location from the optical fibre.

The bone reinforcement structure may be a fixation plate. Fixation plates are attached directly to the bone to span the fracture. The bone reinforcement structure may be an intramedullary nail. An intramedullary nail is placed within the fractured bone along a lengthwise or major axis of the bone such that the nail spans the fracture. The bone reinforcement structure may be an external fixator, wherein at least a portion of the sensing device is disposed outside the patient's body.

The sensing device may be capable of measuring a maximum strain of no less than 2%, preferably at least 10%. This can allow small strains, for example up to 2% during direct bone healing with a fixation plate, to be monitored as well as larger strains, for example between 2% and 10% with secondary bone healing with an intramedullary nail.

The reading may be indicative of temperature, wherein the optical sensor is capable of measuring temperatures between around 25 °C and 50 °C, preferably between 35 °C and 40 °C. This can allow the patient's internal temperature to be measured, for example in order to monitor their general health or to identify potential infection. The reading may be indicative of stress and/or pressure.

The optical fibre may be multimodal. The optical fibre may comprise a plurality of Bragg gratings, preferably wherein each Bragg grating is configured to reflect light with a different range of wavelengths. This can allow measurements to be taken at a plurality of different positions along a single optical fibre. The plurality of Bragg gratings may be disposed along the length of the optical fibre with uniform spacings therebetween. This can provide more information regarding the distribution of strain along the structure and may be used to determine more accurately the load borne by the structure.

The method may further comprise the steps of maintaining the power supply arrangement in a low power state in which substantially no power is provided to the optical sensor.

The signal to trigger powering the optical sensor may be wirelessly transmitted directly from the first communication interface to the processor via the transceiver. The signal encoding the derived measurement may be wirelessly transmitted directly from the processor via the transceiver to the first communication interface. The step of wirelessly transmitting the signal to trigger powering the optical sensor may be performed once the patient has adopted the predetermined pose. Prior to wirelessly transmitting the signal to trigger powering the optical sensor, the method may further comprise the step of determining whether the patient has adopted the predetermined pose.

The method may further comprise the step of pausing for a predetermined length of time after providing the alert before wirelessly transmitting the signal to trigger powering the optical sensor. The step of pausing may comprise the step of providing a countdown to the user to indicate that the user device is about to send the signal to trigger powering the optical sensor.

The method may further comprise the step of receiving a signal indicating that the patient is in the predetermined pose. The step of wirelessly transmitting the signal to trigger powering the optical sensor may be performed after receiving the signal indicating that the patient is in the predetermined pose.

The method may further include the step of deriving a plurality of measurements indicative of strain over a short time interval. The plurality of measurements may comprise at least two, preferably at least three measurements, and the short time interval may comprise a window of 5 seconds or less. The method may further comprise the step of determining that the measurement data is stable **if** each of the plurality of measurements is within a threshold, preferably of less than 5%, more preferably of less than 1%. The method may further comprise the step of indicating a fault or that a measurement should be retaken **if** any of the plurality of measurements is outside the threshold.

The method may further comprise the step of sending a signal to the server to indicate an apparatus fault **if** successive measurements are outside a threshold consistent with estimated likely healing.

The method may further comprise the step of receiving power externally at the power supply arrangement, preferably inductively using an energetic signal. The energetic signal may be transmitted for a duration of around 2 seconds or less, preferably around 1 second, and may have a power of around 500 µW or less.

The method may further comprise the step of wirelessly transmitting the energetic signal from the user device to the sensing device.

The method may further comprise the step of positioning a power device externally to the patient, preferably proximate to the patient's skin, more preferably on the patient's skin near the fractured bone. The method may further comprise the step of wirelessly transmitting the energetic signal from the power device to the sensing device. The step of wirelessly transmitting the energetic signal from the power device to the sensing device may be performed in response to receiving from the first communication interface the signal to trigger powering the optical sensor or an activation signal. The signal to trigger powering the optical sensor may be wirelessly transmitted from the first communication interface via the power device then via the transceiver to the processor. The signal encoding the derived measurement may be wirelessly transmitted from the processor via the transceiver then via the power device to the first communication interface.

The step of powering the optical sensor may be performed for a predetermined length of time, preferably no more than 10 ms, more preferably around 1 ms.

The method may further comprise the step of determining a healing condition of the fracture based on the measurement data. The step of determining the healing condition may comprise the step of comparing the measurement data to one or more thresholds, one or more reference signals, one or more calibration charts and/or one or more calibration datasets. The one or more thresholds may include a value one standard deviation away from a mean value of a sample of reference data. This can allow transmitted measurement data to be used to analytically or empirically determine a healing condition, such as a level of calcification of a bone fracture.

Calibration charts and/or calibration datasets may be based on one or more reference loading curves for a reference bone, preferably the reference bone being the same type as the fractured bone. The reference loading curves may indicate how the strain in the reference bone varies under a plurality of loading conditions. Each loading condition may be created by applying incremental loads to the reference bone until the reference bone fractures. Each loading condition may represent one or more loads, one or more reference bone orientations, and/or one or more reference bone properties. The reference bone properties include at least one of a diameter, a length, a shape and a porosity.

Determining the healing condition may comprise the steps of training a learning engine using a training dataset, providing input data including the measurement data to the learning engine, and receiving as output from the learning engine the healing condition, preferably a classification or a value indicative of treatment progress. The input data may further comprise at least one of data relating to physician availability, the patient's medical history, and population healing data. The method may further comprise the step of indicating, based on the healing condition being above a threshold, that a treatment of the fractured bone is advancing normally or abnormally. The method may further comprise the step of determining a treatment decision using the learning engine based on at least a portion of the input data and/or the healing condition. The step of determining the treatment decision may be performed such that the learning engine assigns a priority to the patient based on the healing condition, physician availability, clinical equipment availability and/or other patient data. The use of the learning engine in this manner can allow powerful databased learning methods such as machine learning or deep learning to be used to determine a healing condition from the transmitted measurement signal as well as other data such as medical histories, survey responses and/or images. Accordingly, patients can be stratified based on expected treatment progression and predicted healing conditions, allowing healthcare resources to be used effectively for better treatment of fractures.

The method may further comprise the steps of determining a second pose for the patient to adopt based on the healing condition, sending instructions on the second pose from the server to the user device, and updating the alert such that it indicates that the patient should adopt the second pose, wherein wirelessly transmitting the signal to trigger powering the optical sensor is performed once the patient has adopted the second pose. Prior to wirelessly transmitting the signal to trigger powering the optical sensor, the method may comprise the step of determining whether the patient has adopted the second pose.

The method may further comprise the steps of deriving a measurement indicative of temperature, and determining a health status based on the derived measurement. The health status may be indicative of local infection, inflammation, a fever, or overheating of components in the sensing device. This can allow the general health of the patient to be monitored, particularly for any symptoms or conditions caused as a result of implanting the sensing device. The method may further comprise the step of deriving a measurement indicative of stress and/or pressure.

Any system feature as described herein may also be provided as a method feature, and vice versa. As used herein, means plus function features may be expressed alternatively in terms of their corresponding structure.

Any, some and/or all features in one aspect of the invention may be applied to any, some and/or all features in any other aspect of the invention, in any appropriate combination.

In particular, method aspects may be applied to system aspects, and vice versa.

It should also be appreciated that particular combinations of the various features described and defined in any aspects of the invention can be implemented and/or supplied and/or used independently.

### BRIEF DESCRIPTION OF THE FIGURES

Methods and systems for monitoring strain on an internal bone of an animal are described by way of example only, in relation to the figures, wherein:
Figure 1a shows a schematic diagram of an example system for monitoring strain on a fractured internal bone;
Figure 1b shows a schematic diagram of an example sensing device in the system of Figure 1a;
Figure 1c shows a schematic diagram of another example sensing device in the system of Figure 1a;
Figures 1d to 1f show schematic diagrams of example communication configurations in the system of Figure 1a;
Figure 2a shows a perspective view of an example bone reinforcement structure;
Figure 2b shows a schematic diagram of the system of Figure 1 ain use with a sensing device implanted in a patient;
Figure 2c shows a fracture fixation which demonstrates the optical fibre laid on the side of the plate;
Figure 2d shows the details of the attachment of the fibre to the PIC;
Figure 2e shows a schematic of the fibre to the surface grating of a PIC interaction;
Figure 3a shows a schematic diagram of an example method for monitoring strain; and
Figure 3b shows a schematic diagram of another example method for monitoring strain.

### DETAILED DESCRIPTION OF EMBODIMENTS

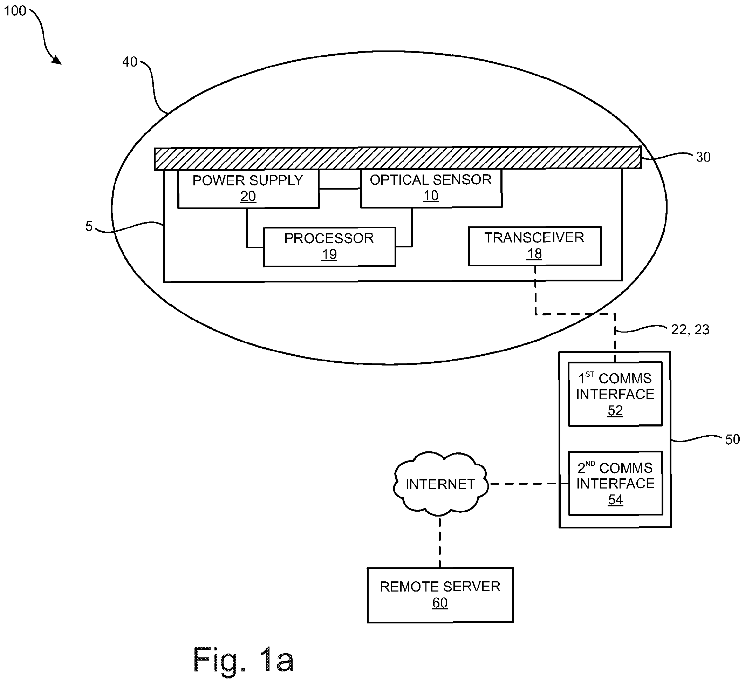

Referring to Figures 1a-b, a system 100 for monitoring strain on an internal bone of an animal will now be described. A sensing device 5 for implanting in a patient 40 includes an optical sensor 10, a power supply arrangement 20, a transceiver 18 and a processor 19. The sensing device 5 is coupled to a bone reinforcement structure 30 to be implanted in the patient 40, with at least the optical sensor 10 being also directly coupled to the structure 30. The structure 30 may be a surgical implant such as a fixation device for treating fractures. The components 10, 18, 19, 20 of the sensing device 5 are jointly encapsulated by a biocompatible coating such as a silicone adhesive which also bonds the device 5 to the structure 30. The power supply arrangement 20 is arranged to provide power to the optical sensor 10 via a wired connection. The processor 19 is communicably linked to the transceiver 18, the power supply arrangement 20 and the optical sensor 10, and is configured to control the power supply arrangement 20 providing power to the optical sensor 10, to receive signals from the optical sensor 10, and receive data from and send data to the transceiver 18.

The processor 19 is configured to communicate wirelessly with a user device 50 located externally to the body of the patient 40. Such communication is facilitated by wireless data exchange between the transceiver 18 and a first communication interface 52 of the user device 50 using a predetermined protocol. The predetermined protocol may be near-field communication (NFC) but is preferably Bluetooth^{®} Low Energy (BLE). The user device 50 can be a smartphone, and the first communication interface may be a BLE interface of the smartphone 50.

The smartphone 50 also has a second communication interface 54, typically a WiFi^{®}, 3G, 4G or 5G interface. The smartphone is configured to communicate with a remote server 60 over the Internet via, for example, its Wi-Fi^{®} interface 54.

The optical sensor 10 includes an optical fibre 12 which has a core and cladding surrounding the core, the cladding may be biocompatible. A light source 14 is optically coupled to the optical fibre 12 at a first end of the fibre 12. A light detector 16 is optically coupled to the optical fibre 12 at a second end of the fibre 12 opposite from the first end.

The power supply arrangement 20 is electrically connected to the light source 14 and is configured to provide power to the light source 14 such that the light source 14 emits light.

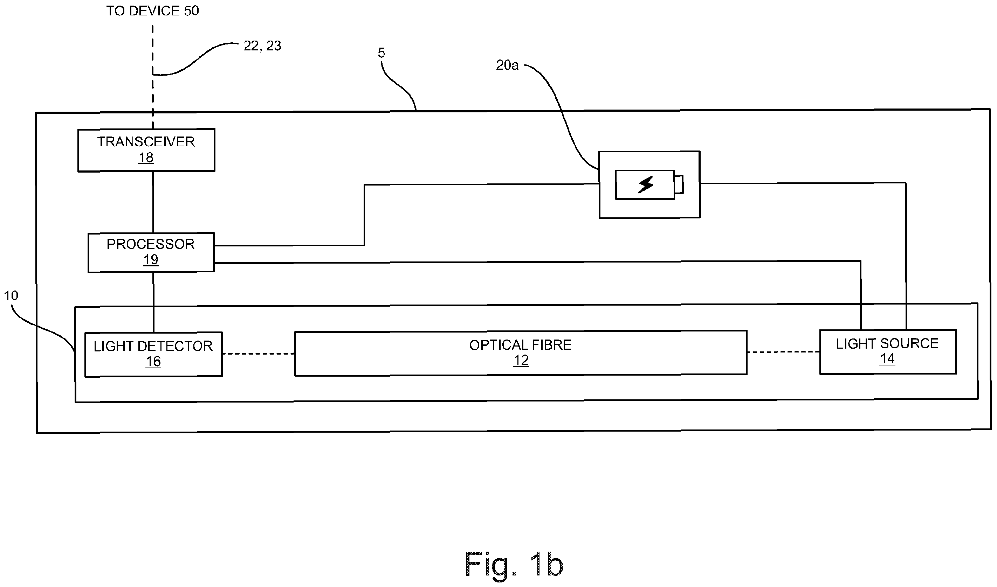

In the example shown in Figure 1b, the power supply arrangement 20 is a battery 20a, such as a button cell battery. The power supply from the battery 20a to the light source 14 can be reversibly connected and disconnected by the processor 19. The transceiver 18 is capable of transmitting and receiving signals wirelessly through bodily tissues such as muscle, fat and skin to and from locations external to the animal's body. Thus, the optical sensor 10 can communicate wirelessly with the device 50.

Ordinarily, the optical sensor 10 and the power supply arrangement 20 are maintained in a low power state. In the low power state, the power supply arrangement 20 is disconnected from the light source 14 such that no power is provided to the light source 14. The processor 19 is capable of receiving signals such as a trigger signal 22 via the transceiver 18 in the low power state. The trigger signal 22 is received wirelessly from the smartphone 50 and is a signal to trigger powering the optical sensor 10. The processor 19 can perform a check, for example using an identification number or an address, that the trigger signal 22 originates from a device authorised to power up the optical sensor 10. Upon successful receipt of the trigger signal 22, the processor 19 switches the optical sensor 10 and the power supply arrangement 20 to a higher power state, causing power to be provided to the light source 14. In some examples, other types of signals may be received from the smartphone 50 such as requests for a status, such as a state of charge of the power supply arrangement 20, or calibration information.

Upon receiving power, the light source 14 transmits light through the optical fibre 12 using one or more laser diodes. The light source 14 transmits light for a predetermined length of time, for example 1 millisecond. This transmitted light travels through the optical fibre 12 and is incident on the light detector 16. As a result of conditions local to the optical fibre 12 causing the optical fibre 12 to deform and/or undergo alteration to its optical properties, the transmitted light is transformed by the optical fibre 12 such that light incident on the light detector 16 is different to that emitted by the light source 14. The light incident on the detector 16 may be the same as the emitted light or a reference light signal if no deformation or change in optical properties of the optical fibre 12 has occurred. The local conditions can include strain, temperature, stress and pressure, but others may also be present.

The light detector 16 converts the incident light into a signal which is provided to the processor 19. The processor 19 uses this signal to derive a measurement indicative of the strain (or any other local condition) on the structure 30. A signal 23 encoding the derived measurement is then transmitted via the transceiver 18 and is received by the smartphone 50.

Following transmission of the signal 23, the optical sensor 10 and the power supply arrangement 20 may wait for a predetermined length of time before returning to the low power state, the processor 19 disconnecting the power supply to the light source 14. In other examples, the optical sensor 10 and the power supply arrangement 20 wait for a stop signal to be received from the smartphone 50 before returning to the low power state. The optical sensor 10 and the power supply arrangement 20 can perform a check, for example using an identification number or address, that the device sending the stop signal is the same device which sent the trigger signal 22 or is the linked to the same user which sent the trigger signal 22.

Where a battery 20a is used, the processor 19 may create a log of readings from the optical sensor 10 by providing power to the optical sensor 10 at predetermined intervals and storing received readings in the log in a memory. The processor 19 will transmit the log of readings to the first communication interface 52 upon receiving a request signal from the first communication interface 52. The request signal may be at least part of a trigger signal 22. The predetermined intervals for creating the log of readings cause readings to be taken from the optical sensor 10 at least weekly and at the same time of day. For example, one or more readings may be taken and stored in the log in memory weekly during the night or at another time of day when the pose of the patient 40 is predictable. The log of readings may be represented as a time series dataset.

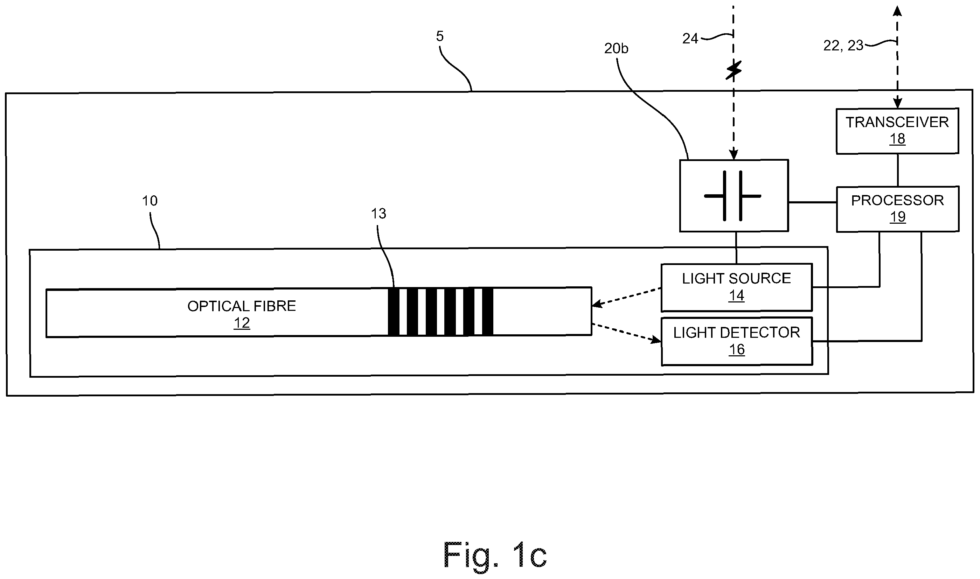

Referring to Figure 1c, another the example sensing device 5 will now be described. In this example, the power supply arrangement 20 is a capacitive element 20b and can include one or more capacitors and an inductive loop or antenna for receiving power wirelessly. The capacitive element 20b receives power wirelessly using an energetic signal 24 received by the processor 19 via the transceiver through the skin of the patient 40. The energetic signal 24 is preferably a radio-frequency signal which causes the capacitive element 20b to charge inductively. The energetic signal 24 can last for a predetermined length of time such that a predetermined amount of energy is stored in the power supply arrangement 20. This predetermined length of time may be longer than that for which the light source 14 is required to emit light, allowing the energetic signal 24 to be of relatively low power. Typically, the processor 19 does not receive the trigger signal 22 until the energetic signal 24 has completed providing the predetermined amount of energy. In this example, the energetic signal 24 is transmitted by the smartphone 50 using in-built apparatus for transmitting power inductively. This allows a single user device 50 to be used to obtain measurements.

A portion of the optical fibre 12 comprises a Bragg grating 13, wherein the refractive index of the optical fibre 12 varies periodically. When provided with power, the light source 14 emits a spectrum of light, for example in infrared, visible or ultraviolet ranges. The Bragg grating 13 is tuned such that it reflects a specific portion the emitted spectrum. A Bragg grating 13 such as this may be used in the optical sensor 10 of this and other example sensing devices 5.

In the example of Figure 1c, the light detector 16 is positioned at the same end of the optical fibre 12 as the light source 14, meaning the light reflected by the Bragg grating 13 is incident on the detector 16. Alternatively or additionally, a light detector 16 can be positioned at the opposite end of the optical fibre 12 from the light source 14 (as shown in Figure 1b to receive the complement of the reflected light transmitted through the optical fibre 12.

When the optical fibre 12 undergoes a strain or a change in temperature, it deforms and so its optical properties change. As a result, the optical properties of the Bragg grating 13 change, meaning the reflected portion of the emitted spectrum changes. Such a change may constitute a change in wavelength of the reflected portion. The change in the reflected portion of light is therefore indicative of the strain or temperature change experienced by the optical fibre 12. The optical fibre 12 may be multimodal and/or may comprise more than one Bragg grating 13, each Bragg grating tuned to reflect a different portion of the emitted light spectrum. Thus, the strain and/or temperature at different points along the optical fibre 12 may be measured.

By having the light source 14 and the light detector 16 at the same end of the optical fibre 12, the spatial distribution of the sensing device 5 components 10, 18, 19, 20 and circuitry can be made more concentrated. For example, the light source 14, light detector 16, transceiver 18, processor 19, power supply arrangement 20 and other associated circuitry can be clustered at one end of the optical fibre 12.

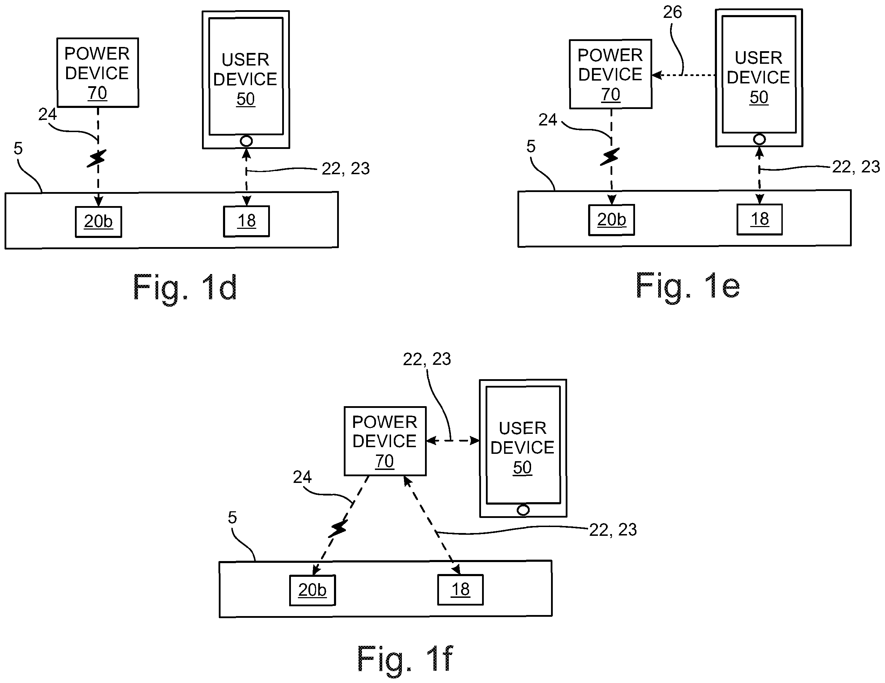

The smartphone 50 is configured to transmit the signal 23 encoding the derived measurement to the server 60, where decisions can be made based on the signal 23. The decisions can include a healing condition of a fracture and/or a treatment decision such as scheduling a scan or prescribing physiotherapy. The decisions, or instructions based on the decisions, are transmitted from the server 60 to the smartphone 50. The smartphone 50 can communicate with the server 60 using a combination of wide area networks (WAN) and local area networks (LAN), which may be wired or wireless. Referring to Figures 1d to 1f, example configurations of the system 100 with the sensing device 5 having a power supply arrangement 20b configured to receive power externally will now be described. In a first example, as shown in Figure 1d, the user device 50 may communicate with the transceiver 18 of the sensing device 5 directly preferably using radio-frequency communication, sending the trigger signal 22 and receiving the signal 23 encoding a derived measurement. A separate power device 70 is used to couple with the power supply arrangement 20b and provide the energetic signal 24 using a radio-frequency transceiver. The power device 70 is arranged to provide a predetermined energetic signal 24 and is activated manually by a user. The processor 19 may provide an indication to the user device 50 via the transceiver 18 that the power supply arrangement 20 is sufficiently charged and that the power device 70 may be deactivated. Using a separate power device allows a user device 50 to be used even if it lacks apparatus for transmitting power inductively.

In a second example, as shown in Figure 1e, the user device 50 can communicate with the power device 70 using radio-frequency signals and/or a low-power communication protocol such as BLE or NFC. Using this protocol, the user device 50 sends a signal 26 to activate or deactivate the power device 70 so as to control the emission of the energetic signal 24. This allows the system 100 to be operated such that a single user device 50 is used to obtain measurements.

In a further example, as shown in Figure 1f, the user device 50 communicates directly with the power device 70 but no longer with the sensing device 5. The power device 70 is arranged to communicate with the user device 50 and with the processor 19 via the transceiver 18, as well as being arranged to couple with the power supply arrangement 20b to transmit the energetic signal 24. The power device 70 is capable of receiving the trigger signal 22 from and sending the signal 23 encoding the derived measurement to the user device 50 using a low-power communication protocol, such as BLE or NFC. The power device 70 is capable of receiving the signal 23 from and sending the trigger signal 22 to the transceiver 18 using the same or a different low-power communication protocol, such as BLE or NFC. The power device 70 is configured to transmit the energetic signal 24 in response to receiving the trigger signal 22 from the user device 50. Once the energetic signal 24 has completed inductively charging the power supply arrangement 20b, the power device 70 sends the trigger signal 22 to the processor 19 via the transceiver 18. In this example, all signals sent to and received from the sensing device 5 pass through the power device 70, meaning the transceiver 18 and the power supply arrangement 20b need only receive or send wireless signals to the exterior of the patient's skin, rather than through potentially much greater additional distances through air to the user device 50 directly.

In the examples of Figures 1d to 1f, in operation, the power device 70 is held against the skin of the patient 40 such that the transmission medium between the power device 70 and the sensing device 5 substantially consists of bodily tissues of the patient 40.

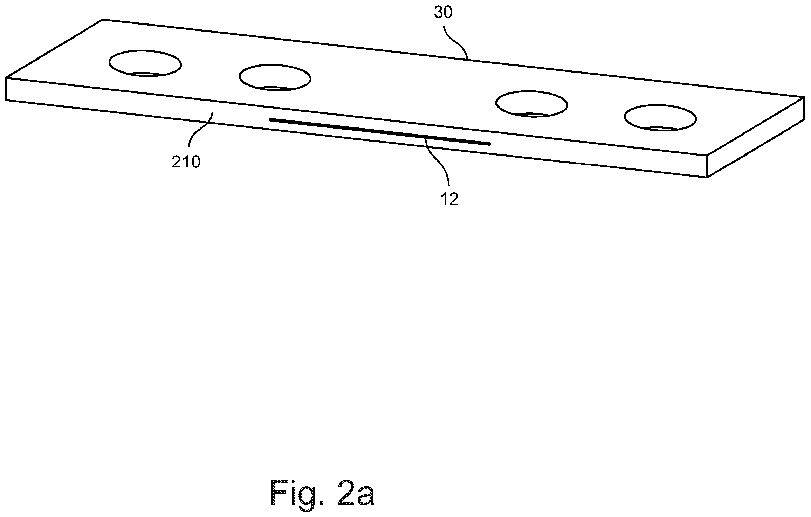

Referring to Figure 2a, an example an optical sensor 10 in the system 100 for monitoring healing of a fracture using a bone reinforcement structure 30 will now be described. In this implementation, the system 100 is used to obtain measurement signals indicative of strain periodically over the course of a fracture treatment process. An optical sensor 10 is coupled to the structure 30. The structure 30 is a fixation device for attaching to bone either side of a fracture. The sensing device 5 (not shown) is coupled to the fixation device 30 with a biocompatible bonding material, such as acrylic (polymethyl methacrylate) or a silicone. The bonding material attaches the sensing device 5 to the fixation device 30 by bonding at least the optical fibre 12 to a surface 210 of the fixation device 30 such that at least the optical fibre 12 is stationary relative to the surface 210. The surface 210 can be a side surface (as shown in Figure 2a), or a top surface of the fixation device 30. The surface 210 may have a width of no more than 4.5 mm. Other components of the sensing device 5 may also be disposed on the surface 210 or may be disposed at other locations.

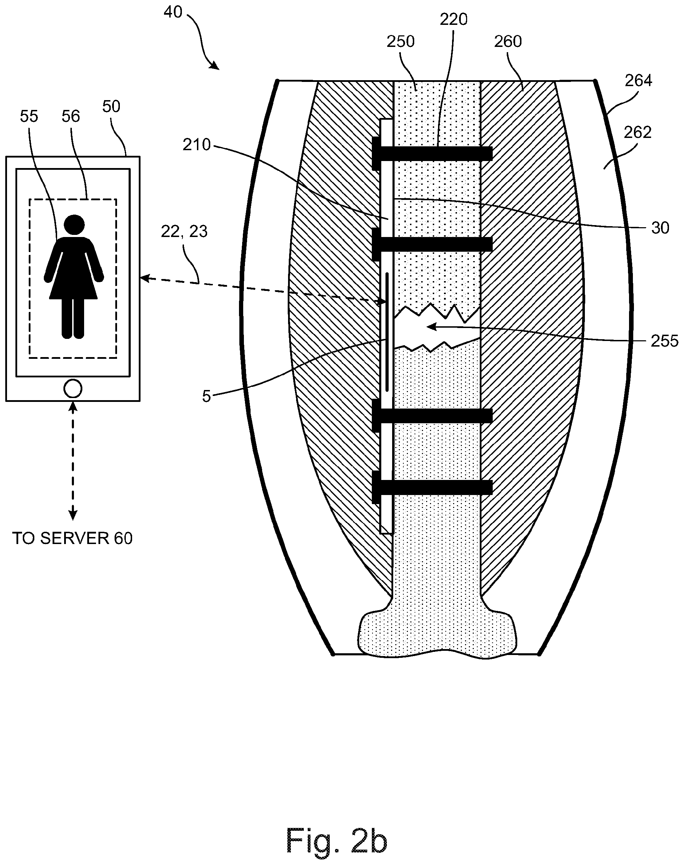

Referring to Figure 2b, an example implementation of the system 100 implanted in a patient 40 for treating a fracture 255 will now be described. The fixation device 30 with the sensing device 5 attached is implanted in the patient 40 having a fracture 255 to an internal bone 250. Typically, the fixation device 30 is fixed to the internal bone 250 using pins 220 which penetrate into the bone 250 such that the fixation device 30 bridges the site of the fracture 255 while the fracture 255 heals. Other types and shapes of fixation device can be used for different types of fractures, bones and/or animals.

The sensing device 5 can be used to monitor the amount of strain in the fixation device 30. Each time a strain measurement is required, the user device 50 provides an alert 55 on an alerting device 56 to the user to indicate that the patient 40 should adopt a predetermined pose. This provides a controlled loading environment for which to measure strain. The alert 55 is preferably a graphic or an animation providing instruction and/or depicting the pose which the patient 40 is required to adopt. Alternatively or additionally, the alert 55 may be a textual instruction, a change in appearance of a light or icon, an audio instruction, or a haptic indication from an electromechanical component within the user device 50.

The sensing device 5 or another sensor implanted or external to the patient 40 can determine that the patient 40 has adopted the predetermined pose. Once the patient 40 has adopted the predetermined pose, the user device 50 transmits the trigger signal 22 to the sensing device 5, and in response receives from the sensing device a signal 23 encoding a derived measurement indicative of the strain in the fixation device 30. Communication is performed wirelessly between the sensing device 5 and the user device 50 such that transmitted signals 22, 23 are able to permeate bodily tissue of the patient 40 such as bone 250, muscle 260, fat 262 and/or skin 264 such that little or no data is lost.

The signal 23 can be used to infer the healing progress of the fracture 255. Initially, in the case of a total fracture of the bone 250, the fixation device 30 will bear the entirety of a load applied between the ends of the bone 250. As the fracture 255 starts to heal, the bone 250 bears more and more of the load, meaning the load in the fixation device 30 reduces, and so too does the amount of strain and stress. The sensing device 5 can be calibrated for the patient 40, the type of bone 250, the type of fracture 255 and/or the type of structure 30, and the bone 250 can be loaded in a controlled manner. Measurement data based on the signal 23 can then be sent to the server 60 to be compared with known values, thresholds and/or calibration charts associated with different stages of healing in order to determine a healing progress of the fracture 255.

In other examples, a learning engine is used to determine a healing condition based on the measurement data. The learning engine receives the measurement data as input and outputs a healing condition, such as an expected level of calcification of the bone 250, a predicted recovery time, or a classification indicating the level of healing. Other data such as physician availability, the patient's medical history, and population healing data are also available to the learning engine as input. The learning engine is trained using a training dataset. The healing condition may be interpreted by a clinician who may predict when the fracture 255 will heal and/or may determine a subsequent stage of treatment. Alternatively or additionally, the learning engine may determine a subsequent stage of treatment automatically, such as scheduling a scan, scheduling a remote consultation, or prescribing physiotherapy.

The healing condition or an instruction relating to a treatment decision is transmitted from the server 60 to the user device 50. If the healing condition or instruction indicates that healing of the fracture 255 is progressing, based on the healing condition or instruction the alerting device 56 updates the alert 55 such that it indicates that the patient 40 should adopt a second pose. For example, if previous measurement signals were taken while the animal 40 was in a sitting position or while applying loads to the bone 250 less than its body weight, the healing condition may indicate that sufficient healing has occurred across the fracture 255 to allow the animal 40 to stand or provide higher loading to the bone 250 when obtaining measurement signals. This can accelerate the healing process and can allow the duration of healing to be predicted more accurately. Upon determining that the patient 40 has adopted the second pose, the user device 50 transmits the trigger signal 22 causing a further signal 23 encoding a derived measurement indicative of strain to be obtained.

In the examples of Figures 2a and 2b, the optical sensor 10 may also be used to obtain measurements indicative of temperature, for example using a multimodal fibre or a fibre with multiple Bragg gratings. The signal 23 may include temperature information as well as or instead of strain information, allowing a learning engine or a remote clinician to monitor the internal temperature local to the site of the fracture 255, for example to determine a health status such as a local infection, inflammation or a fever, or whether any components of the sensing device 5 are overheating.

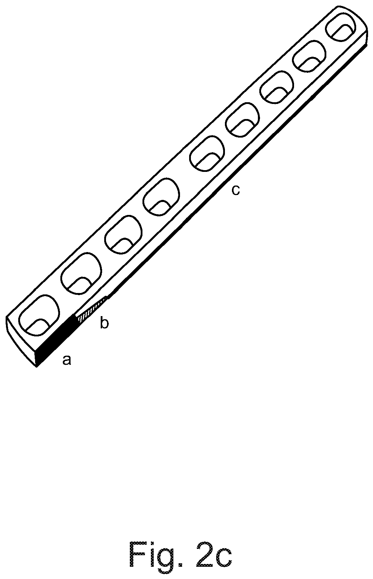

Fig. 2c shows the optical fibre 12 laid on the side 210 of the plate 30 in further detail.

Specifically, in Fig 2c, there are three portions shown, labelled as a, b, and c.
a= Indium Phosphide PIC (photonic integrated circuit)
b= Coupling mechanism of PIC to fibre optic cable
c= FBG (Fibre Bragg Grating) optical fibre cable

In this way, there is a direct coupling to an Indium Phosphide Photonics Integrated Circuit (PIC) designed from Indium Phosphide. Preliminary investigations have shown that Indium Phosphide as a material provides the best means of achieving a reduction in size of the circuit and reduces the power needs throughout the use of the device. The device for fracture monitoring will be required for at least 6 months.

The PIC will house the laser for transmission of the light through the FBG (Fibre Bragg Grating) fibre and the sensor for measuring the change in strain and transmission of the data externally to a device outside the body, wirelessly. The use of Indium Phosphide (InP) as the guiding platform for the laser light simplifies the integration of both passive and active functions in the circuit.

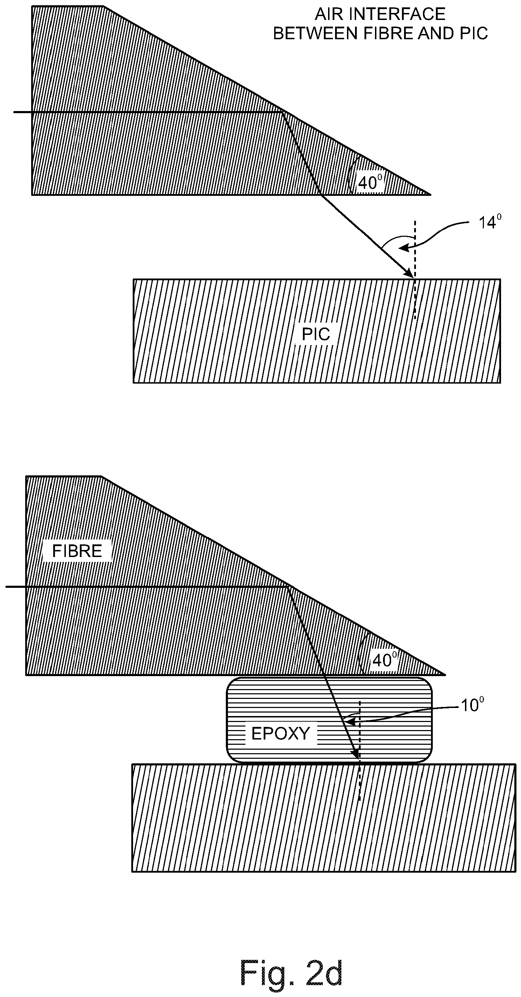

Fig 2d shows the details regarding the attachment of the fibre 12 to the PIC. The arrow shows the path of the light coming into the PIC.

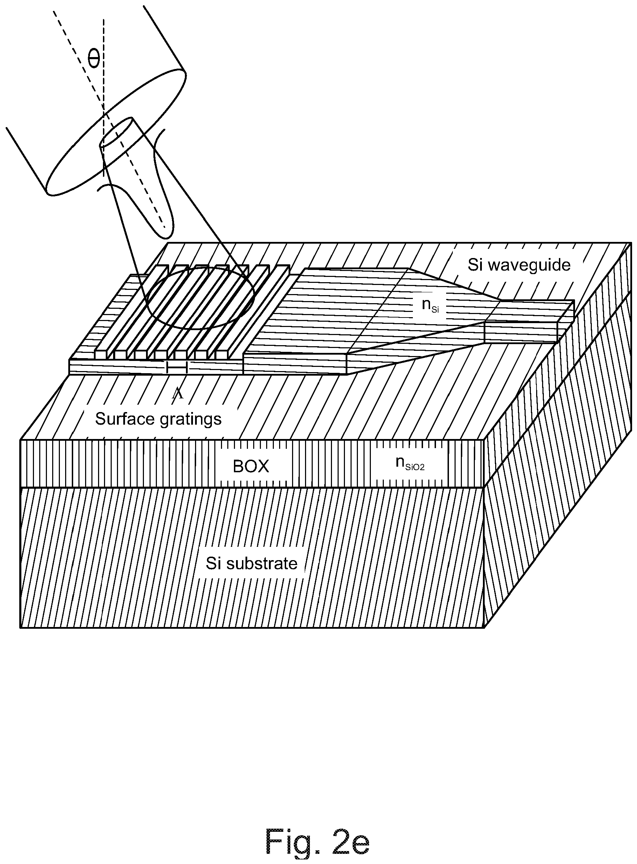

Fig 2e shows the interaction between the light that is emitted by the fibre and that is then guided by the surface grating on the surface of the PIC. In this case the diagram shows a silicon based PIC whereas the preferred PIC would be an Indium Phosphide composite.

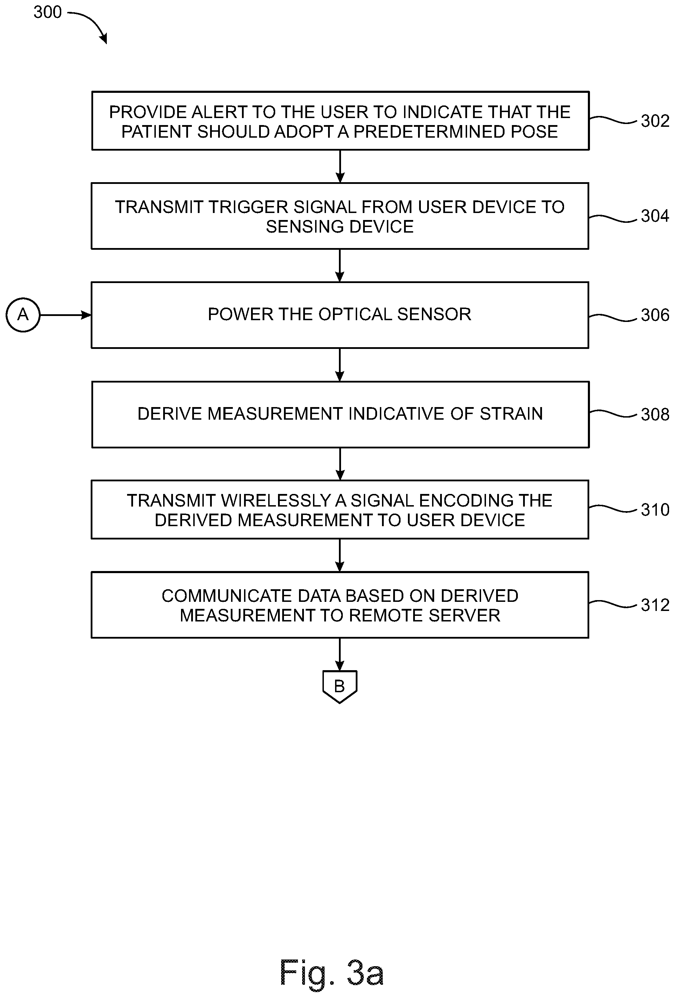

Referring to Figure 3a, an example method 300 for monitoring strain on an internal bone 250 in a patient 40 will now be described. Using a system such as the system 100 described above, the method 300 includes the step of providing 302 on a user device 50 an alert to the user to indicate that the patient 40 should adopt a predetermined pose. After determining that the patient 40 has adopted the predetermined pose, receiving an indication such as through a button on the user device 50 that the patient 40 has adopted the predetermined pose, or waiting for a predetermined length of time, a trigger signal 22 is transmitted 304 from the user device 50 to a sensing device 5 implanted in the animal 40. This causes the power supply arrangement 20 to power 306 an optical sensor 10 in the sensing device 5. Powering 306 the optical sensor 10 may include transmitting an energetic signal 24 to the sensing device 5 if the power supply arrangement 20 is configured to receive power externally. If the power supply arrangement 20 comprises a power source such as a battery, a processor 19 in the sensing device 5 may connect the power source to the optical sensor 10. In powering 306 the optical sensor 10, a light source 14 within the optical sensor 10 is provided with power and begins to emit light through an optical fibre 12 and onto a light detector 16.

A signal is produced by the light detector 16, from which the processor 19 derives 308 a measurement indicative of strain and/or temperature. The measurement is encoded in a signal 23 which is transmitted 310 wirelessly via a short-range wireless transceiver 18 to the user device 50.

The signal 23 is received wirelessly at the device 50 and data based on the derived measurement is communicated 312 to a remote server 60 for determining a healing condition.

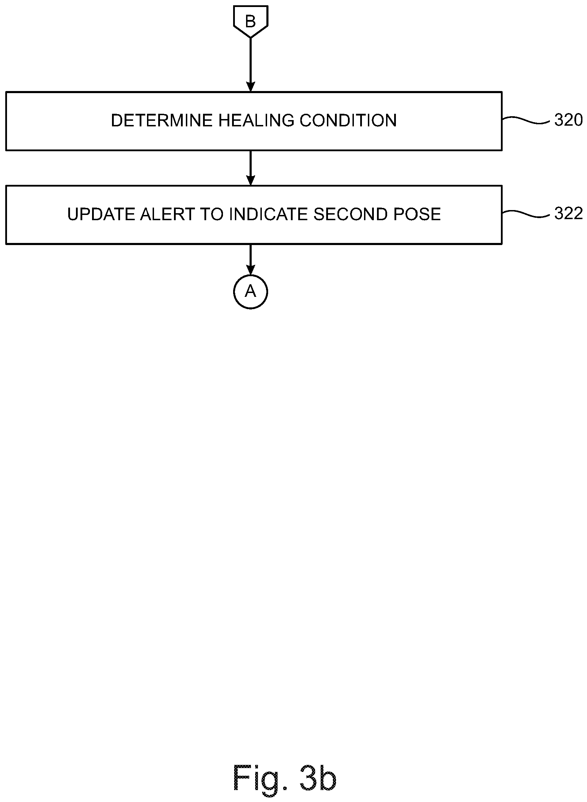

Referring to Figure 3b, a further example of the method 300 for monitoring strain on an internal bone 250 in a patient 40 will now be described. Once the remote server 60 receives the data based on the derived measurement, a healing condition is determined 320. The healing condition or an instruction based on a treatment decision may be transmitted directly to the user device 50 from the server 60. Based on the healing condition or the instruction, the alerting device 56 updates 322 the alert 55 such that it indicates that the patient 40 should adopt a second pose. Upon determining that the patient 40 has adopted the second pose, receiving an indication such as through a button on the user device 50 that the patient 40 has adopted the predetermined pose, or waiting for a predetermined length of time, steps 306 to 312 (shown in Figure 3a) are performed in order to obtain a measurement indicative of strain or temperature.

While specific systems and sensors are shown, any appropriate hardware/software architecture may be employed. For example, communication between sensor components may be via optical or wireless connections.

The above embodiments and examples are to be understood as illustrative examples. Further embodiments, aspects or examples are envisaged. It is to be understood that any feature described in relation to any one embodiment, aspect or example may be used alone, or in combination with other features described, and may also be used in combination with one or more features of any other of the embodiments, aspects or examples, or any combination of any other of the embodiments, aspects or examples. The scope of the invention is defined in the accompanying claims.

## Claims

1. A system (100) for monitoring strain on a musculoskeletal element of a patient (10) having an injury at an injury location, arranged to perform monitoring at least once a week, over a period of at least 12 weeks, or at least 12 times, the system comprising:
an implantable load-bearing fixation and sensing device comprising:
an implantable reinforcement structure (30);
a deformation sensing arrangement (10) physically coupled to the reinforcement structure (30) to be at least partially implanted in the patient, the deformation sensing arrangement (10) for sensing deformation of the implantable reinforcement structure (30);
a power supply arrangement (20) arranged to provide power to the deformation sensing arrangement (5);
a short-range wireless transceiver (18) arranged to communicate digital data using a first protocol; and
a processor (19) arranged to control the power supply arrangement (20) to power the deformation sensing arrangement (10) selectively, to obtain a measure indicative of deformation associated with stress or movement in the vicinity of the injury location, to communicate via the short range wireless transceiver (18) to an external user device (50), to receive a first signal to trigger powering the deformation sensing arrangement (10) and to transmit a second signal based on said measure to the external user device (50) ; and the extermal user device (50) comprising:
a first communication interface (52) configured when positioned externally of the patient (40) to send the first signal to trigger the processor (19) to power the deformation sensing arrangement (10) and obtain the measure indicative of deformation in the vicinity of the injury location, and to receive the second signal using the first protocol;
a second communication interface (54) configured to communicate measurement data based on the second signal to a remote server (60); and
an arrangement for triggering a measurement correlated with the patient adopting a predetermined or known pose with respect to the injury location; and
at least one alerting device (56) arranged to provide an audible and/or visual alert (55) to a user to indicate that the patient (40) should adopt the predetermined or known pose, wherein the external user device (50) is configured to determine whether the patient (40) has adopted the predetermined pose using one or more sensors and/or one or more indications from the user that the patient (40) has adopted the predetermined pose and wherein the first communication interface (53) is configured to send the first signal to trigger the processor (19) to power the deformation sensing arrangement (10) once the patient (40) has adopted the predetermined pose.

2. A system according to Claim 1, wherein the implantable load-bearing fixation and sensing device comprises an elongate bone fixation element having a plurality of fixing holes spaced apart wherein the deformation sensing arrangement is arranged for sensing strain at an injury location along the elongate bone fixation element.

3. A system according to Claim 2, wherein the deformation sensing arrangement is arranged to sense strain at a range of locations along the elongate bone fixation element and/or wherein the processor is arranged to determine a representative measure of strain at an injury location.

4. A system according to any preceding claim, wherein the injury location is selectable or adjustable.

5. A system according to any preceding claim, wherein the deformation sensing arrangement comprises a distributed strain sensing arrangement, which may comprise a plurality of strain sensors.

6. A system according to any one of claims 1 to 4, wherein the deformation sensing arrangement comprises an optical fibre, optionally wherein at least a portion of the optical fibre comprises a Bragg grating.

7. A system according to claim 6, wherein a photonic integrated circuit is coupled to the optical fibre, preferably wherein the photonic integrated circuit is an Indium Phosphide integrated circuit.

8. A system according to any preceding claim, wherein the implantable reinforcement structure is elongate and the deformation sensing arrangement is arranged to measure strain across a range of locations along the reinforcement structure, optionally including processing logic for selectively determining a representative measure of strain in the reinforcement structure in the vicinity of an injury location.

9. A system according to any preceding claim, wherein the audible and/or visual alert to the user comprises at least one of a textual and/or audio instruction, a graphic, an animation, a light, or a sound.

10. The system according to any preceding claim, wherein the musculoskeletal element is a fractured internal bone, wherein the sensing device is implantable and encapsulated in a biocompatible housing or coating, and wherein the reinforcement structure is a bone reinforcement structure fully implanted in the patient, preferably , wherein the sensing device is physically coupled to the reinforcement structure using a biocompatible adhesive, preferably a silicone, and wherein the biocompatible adhesive provides the biocompatible housing or coating.

11. The system according to any preceding claim, wherein the user device is provided by an application running on a mobile device, optionally a smartphone, a tablet, a computer or a smart watch.

12. The system according to any preceding claim, wherein the power supply arrangement is arranged to receive power from an external power source, preferably inductively using an energetic signal, optionally wherein the power supply arrangement comprises a capacitor configured to charge inductively using the energetic signal, optionally wherein the energetic signal has a power of around 500 µW or less and is transmitted for a duration of around 2 seconds or less, preferably around 1 second, preferably wherein the energetic signal is a radio-frequency signal, optionally wherein the user device further comprises apparatus for transmitting the energetic signal to power the sensing device inductively or wherein the system further comprises a power device for transmitting the energetic signal to power the sensing device inductively, optionally wherein the processor is arranged to communicate with the user device via the power device.

13. The system according to any preceding claim, wherein the processor is configured to power up the sensor at predetermined intervals and create a log of readings in a memory, and wherein the processor is configured to transmit the log of readings upon receiving a request from the user device, optionally wherein the power supply arrangement is configured to provide power to the sensor for a predetermined length of time for each measurement, preferably no more than 10 ms, more preferably around 1 ms.

14. The system according to any preceding claim, further comprising the remote server, wherein the remote server is configured to determine a healing condition of the fracture based on the measurement data, optionally wherein, in determining the healing condition, the server compares the measurement data with one or more thresholds and/or one or more reference signals, optionally wherein the server comprises a learning engine trained using a training dataset, the learning engine configured to receive input data including the measurement data and to provide as output the healing condition, preferably a classification or a value indicative of treatment progress, optionally wherein the server is configured to transmit the healing condition to the user device, and wherein the alerting device is arranged, in response to receiving the healing condition, to update the alert such that it indicates that the patient should adopt a second pose, optionally wherein the first communication interface is configured to send the signal to trigger the processor to power the optical sensor once the patient has adopted the second pose.

15. The system according to any one of Claims 7 to 14, when dependent on Claim 6, wherein the optical fibre is multimodal and/or wherein a measurement indicative of a temperature is obtained, optionally wherein the sensor is capable of measuring temperatures between around 25 °C and 50 °C, or preferably covering a range of 35 °C to 40 °C.

## Patentansprüche

1. System (100) zum Überwachen der Belastung eines muskuloskelettalen Elements eines Patienten (10) mit einer Verletzung an einer Verletzungsstelle, das so angeordnet ist, dass es die Überwachung mindestens einmal pro Woche über einen Zeitraum von mindestens 12 Wochen oder mindestens 12 Mal durchführt, wobei das System Folgendes umfasst:
eine implantierbare lasttragende Fixierungs- und Erfassungsvorrichtung, die Folgendes umfasst:
eine implantierbare Verstärkungsstruktur (30);
eine Verformungserfassungsanordnung (10), die physisch mit der Verstärkungsstruktur (30) gekoppelt ist, die mindestens teilweise in den Patienten implantiert werden soll, wobei die Verformungserfassungsanordnung (10) zum Erfassen der Verformung der implantierbaren Verstärkungsstruktur (30) dient;
eine Stromversorgungsanordnung (20), die so angeordnet ist, dass sie der Verformungserfassungsanordnung (5) Strom zuführt;
einen drahtlosen Kurzstrecken-Sender-Empfänger (18), der so angeordnet ist, dass er digitale Daten unter Verwendung eines ersten Protokolls übermittelt; und
einen Prozessor (19), der so beschaffen ist, dass er die Stromversorgungsanordnung (20) steuert, um die Verformungserfassungsanordnung (10) selektiv mit Strom zu versorgen, um eine Messung zu erhalten, die eine Verformung anzeigt, die mit einer Belastung oder Bewegung in der Nähe der Verletzungsstelle verbunden ist, um über den drahtlosen Kurzstrecken-Sender-Empfänger (18) mit einer externen Benutzervorrichtung (50) zu kommunizieren, um ein erstes Signal zu empfangen, um die Stromversorgung der Verformungserfassungsanordnung (10) auszulösen, und um ein zweites Signal auf der Grundlage der Messung an die externe Benutzervorrichtung (50) zu senden; und
die externe Benutzervorrichtung (50), umfassend:
eine erste Kommunikationsschnittstelle (52), die so konfiguriert ist, dass sie, wenn sie außerhalb des Patienten (40) positioniert ist, das erste Signal sendet, um den Prozessor (19) zu veranlassen, die Verformungserfassungsanordnung (10) mit Strom zu versorgen und das Maß zu erhalten, das die Verformung in der Nähe der Verletzungsstelle anzeigt, und um das zweite Signal unter Verwendung des ersten Protokolls zu empfangen;
eine zweite Kommunikationsschnittstelle (54), die so konfiguriert ist, dass sie Messdaten auf der Grundlage des zweiten Signals an einen entfernten Server (60) übermittelt; und
eine Anordnung zum Auslösen einer Messung, die damit korreliert, dass der Patient eine vorbestimmte oder bekannte Haltung in Bezug auf die Verletzungsstelle einnimmt; und
mindestens eine Warnvorrichtung (56), die so angeordnet ist, dass sie eine akustische und/oder visuelle Warnung (55) für einen Benutzer bereitstellt, um anzuzeigen, dass der Patient (40) die vorbestimmte oder bekannte Haltung einnehmen sollte, wobei die externe Benutzervorrichtung (50) so konfiguriert ist, dass sie bestimmt, ob der Patient (40) die vorbestimmte Haltung eingenommen hat, indem sie einen oder mehrere Sensoren und/oder einen oder mehrere Hinweise des Benutzers verwendet, dass der Patient (40) die vorbestimmte Haltung eingenommen hat, und wobei die erste Kommunikationsschnittstelle (53) so konfiguriert ist, dass sie das erste Signal sendet, um den Prozessor (19) zu veranlassen, die Verformungserfassungsanordnung (10) mit Strom zu versorgen, sobald der Patient (40) die vorbestimmte Haltung eingenommen hat.

2. System nach Anspruch 1, wobei die implantierbare lasttragende Fixierungs- und Erfassungsvorrichtung ein längliches Knochenfixierungselement mit einer Vielzahl von Befestigungslöchern umfasst, die voneinander beabstandet sind, wobei die Verformungserfassungsanordnung so angeordnet ist, dass sie die Belastung an einer Verletzungsstelle entlang des länglichen Knochenfixierungselements erfasst.

3. System nach Anspruch 2, wobei die Verformungserfassungsanordnung so angeordnet ist, dass sie die Belastung an einer Reihe von Stellen entlang des länglichen Knochenfixierungselements erfasst und/oder wobei der Prozessor so angeordnet ist, dass er ein repräsentatives Maß der Belastung an einer Verletzungsstelle bestimmt.

4. System nach einem der vorhergehenden Ansprüche, wobei die Verletzungsstelle wählbar oder einstellbar ist.

5. System nach einem der vorhergehenden Ansprüche, wobei die Verformungserfassungsanordnung eine verteilte Belastungserfassungsanordnung umfasst, die eine Vielzahl von Belastungssensoren umfassen kann.

6. System nach einem der Ansprüche 1 bis 4, wobei die Verformungserfassungsanordnung eine optische Faser umfasst, wobei optional mindestens ein Teil der optischen Faser ein Bragg-Gitter umfasst.

7. System nach Anspruch 6, wobei ein integrierter photonischer Schaltkreis mit der optischen Faser gekoppelt ist, wobei der integrierte photonische Schaltkreis vorzugsweise ein integrierter Indiumphosphid-Schaltkreis ist.

8. System nach einem der vorhergehenden Ansprüche, wobei die implantierbare Verstärkungsstruktur länglich ist und die Verformungserfassungsanordnung so angeordnet ist, dass sie die Belastung über einen Bereich von Stellen entlang der Verstärkungsstruktur misst, wobei sie optional eine Verarbeitungslogik zur selektiven Bestimmung eines repräsentativen Maßes der Belastung in der Verstärkungsstruktur in der Nähe einer Verletzungsstelle enthält.

9. System nach einem der vorhergehenden Ansprüche, wobei die akustische und/oder visuelle Warnung an den Benutzer mindestens eine textliche und/oder akustische Anweisung, eine Grafik, eine Animation, ein Licht oder einen Ton umfasst.

10. System nach einem der vorhergehenden Ansprüche, wobei das muskuloskelettale Element ein gebrochener innerer Knochen ist, wobei die Erfassungsvorrichtung implantierbar und in einem biokompatiblen Gehäuse oder einer biokompatiblen Beschichtung eingekapselt ist, und wobei die Verstärkungsstruktur eine vollständig in den Patienten implantierte Knochenverstärkungsstruktur ist, wobei die Erfassungsvorrichtung vorzugsweise physisch mit der Verstärkungsstruktur unter Verwendung eines biokompatiblen Klebstoffs, vorzugsweise eines Silikons, gekoppelt ist und wobei der biokompatible Klebstoff das biokompatible Gehäuse oder die biokompatible Beschichtung bildet.

11. System nach einem der vorhergehenden Ansprüche, wobei die Benutzervorrichtung durch eine Anwendung bereitgestellt wird, die auf einem mobilen Gerät, optional einem Smartphone, einem Tablet, einem Computer oder einer Smartwatch, läuft.

12. System nach einem der vorhergehenden Ansprüche, wobei die Stromversorgungsanordnung so beschaffen ist, dass sie Strom von einer externen Stromquelle erhält, vorzugsweise induktiv unter Verwendung eines energetischen Signals, wobei die Stromversorgungsanordnung optional einen Kondensator umfasst, der so konfiguriert ist, dass er sich unter Verwendung des energetischen Signals induktiv auflädt, wobei das energetische Signal optional eine Leistung von etwa 500 µW oder weniger hat und für eine Dauer von etwa 2 Sekunden oder weniger, vorzugsweise etwa 1 Sekunde, übertragen wird, wobei das energetische Signal vorzugsweise ein Hochfrequenzsignal ist, optional, wobei die Benutzervorrichtung ferner eine Vorrichtung zum Übertragen des energetischen Signals umfasst, um die Erfassungsvorrichtung induktiv mit Strom zu versorgen, oder wobei das System ferner eine Energievorrichtung zum Übertragen des energetischen Signals umfasst, um die Erfassungsvorrichtung induktiv mit Energie zu versorgen, optional, wobei der Prozessor so angeordnet ist, dass er mit der Benutzervorrichtung über die Energievorrichtung kommuniziert.

13. System nach einem der vorhergehenden Ansprüche, wobei der Prozessor so konfiguriert ist, dass er den Sensor in vorgegebenen Intervallen einschaltet und ein Messwertprotokoll in einem Speicher erstellt, und wobei der Prozessor so konfiguriert ist, dass er das Messwertprotokoll nach Erhalt einer Anforderung von der Benutzervorrichtung überträgt, wobei die Stromversorgungsanordnung optional so konfiguriert ist, dass sie den Sensor für eine vorgegebene Zeitspanne für jede Messung mit Strom versorgt, vorzugsweise nicht länger als 10 ms, besser noch etwa 1 ms.

14. System nach einem der vorhergehenden Ansprüche, das außerdem den entfernten Server umfasst, wobei der entfernte Server so konfiguriert ist, dass er auf der Grundlage der Messdaten einen Heilungszustand der Fraktur bestimmt, wobei der Server optional bei der Ermittlung des Heilungszustands die Messdaten mit einem oder mehreren Schwellenwerten und/oder einem oder mehreren Referenzsignalen vergleicht, wobei der Server optional eine Lernmaschine umfasst, die unter Verwendung eines Trainingsdatensatzes trainiert wird, wobei die Lernmaschine so konfiguriert ist, dass sie Eingabedaten einschließlich der Messdaten empfängt und als Ausgabe den Heilungszustand bereitstellt, vorzugsweise eine Klassifizierung oder einen Wert, der den Behandlungsfortschritt anzeigt, wobei optional der Server so konfiguriert ist, dass er den Heilungszustand an die Benutzervorrichtung überträgt, und wobei die Warnvorrichtung so eingerichtet ist, dass sie als Reaktion auf den Empfang des Heilungszustands die Warnung so aktualisiert, dass sie anzeigt, dass der Patient eine zweite Haltung einnehmen sollte, wobei optional die erste Kommunikationsschnittstelle dazu konfiguriert ist, das Signal zum Auslösen des Prozessors zum Einschalten des optischen Sensors zu senden, sobald der Patient die zweite Haltung eingenommen hat.

15. System nach einem der Ansprüche 7 bis 14, sofern abhängig von Anspruch 6, wobei die optische Faser multimodal ist und/oder wobei eine eine Temperatur anzeigende Messung erhalten wird, wobei der Sensor optional in der Lage ist, Temperaturen zwischen etwa 25°C und 50°C zu messen oder vorzugsweise einen Bereich von 35°C bis 40°C abzudecken.

## Revendications

1. Système (100) de surveillance de contrainte sur un élément musculo-squelettique d'un patient (10) présentant une lésion au niveau d'un site de lésion, conçu pour réaliser une surveillance au moins une fois par semaine, sur une période d'au moins 12 semaines, ou au moins 12 fois, le système comprenant :
un dispositif implantable de fixation et de détection porteur comprenant :
une structure de renforcement implantable (30) ;
un agencement de détection de déformation (10) couplé physiquement à la structure de renforcement (30) pour être au moins partiellement implanté dans le patient, l'agencement de détection de déformation (10) étant destiné à détecter une déformation de la structure de renforcement implantable (30) ;
un agencement d'alimentation électrique (20) conçu pour fournir une puissance à l'agencement de détection de déformation (5) ;
un émetteur-récepteur sans fil à courte portée (18) conçu pour communiquer des données numériques à l'aide d'un premier protocole ; et
un processeur (19) conçu pour contrôler l'agencement d'alimentation électrique (20) pour alimenter en puissance sélectivement l'agencement de détection de déformation (10), pour obtenir une mesure indiquant une déformation associée à une tension ou à un mouvement à proximité du site de lésion, pour communiquer via l'émetteur-récepteur sans fil à courte portée (18) à un dispositif utilisateur externe (50), pour recevoir un premier signal pour déclencher l'alimentation en puissance de l'agencement de détection de déformation (10) et transmettre un second signal sur la base de ladite mesure au dispositif utilisateur externe (50) ; et
le dispositif utilisateur externe (50) comprenant :
une première interface de communication (52) configurée lorsqu'elle est positionnée à l'extérieur du patient (40) pour envoyer le premier signal pour déclencher le processeur (19) pour qu'il alimente en puissance l'agencement de détection de déformation (10) et obtenir la mesure indiquant une déformation à proximité du site de lésion, et pour recevoir le second signal en utilisant le premier protocole ;
une seconde interface de communication (54) configurée pour communiquer des données de mesure sur la base du second signal à un serveur distant (60) ; et
un agencement de déclenchement d'une mesure corrélée au patient qui adopte une posture prédéterminée ou connue par rapport au site de lésion ; et
au moins un dispositif d'alerte (56) conçu pour fournir une alerte sonore et/ou visuelle (55) à un utilisateur pour indiquer que le patient (40) doit adopter la posture prédéterminée ou connue, dans lequel le dispositif utilisateur externe (50) est configuré pour déterminer si le patient (40) a adopté la posture prédéterminée à l'aide d'un ou plusieurs capteurs et/ou d'une ou plusieurs indications de l'utilisateur que le patient (40) a adopté la posture prédéterminée et dans lequel la première interface de communication (53) est configurée pour envoyer le premier signal pour déclencher le processeur (19) pour qu'il alimente en puissance l'agencement de détection de déformation (10) une fois que le patient (40) a adopté la posture prédéterminée.

2. Système selon la revendication 1, dans lequel le dispositif implantable de fixation et de détection porteur comprend un élément de fixation osseuse allongé ayant une pluralité de trous de fixation espacés dans lequel l'agencement de détection de déformation est conçu pour détecter une contrainte au niveau d'un site de lésion le long de l'élément de fixation osseuse allongé.

3. Système selon la revendication 2, dans lequel l'arrangement de détection de déformation est conçu pour détecter une contrainte dans une plage de sites le long de l'élément de fixation osseuse allongé et/ou dans lequel le processeur est conçu pour déterminer une mesure représentative de contrainte à un site de lésion.

4. Système selon l'une des revendications précédentes, dans lequel le site de lésion est sélectionnable ou réglable.

5. Système selon l'une des revendications précédentes, dans lequel l'agencement de détection de déformation comprend un agencement de détection de contrainte distribué, qui peut comprendre une pluralité de capteurs de contrainte.

6. Système selon l'une des revendications 1 à 4, dans lequel l'agencement de détection de déformation comprend une fibre optique, facultativement dans lequel au moins une partie de la fibre optique comprend un réseau de Bragg.

7. Système selon la revendication 6, dans lequel un circuit intégré photonique est couplé à la fibre optique, de préférence dans lequel le circuit intégré photonique est un circuit intégré de phosphure d'indium.

8. Système selon l'une des revendications précédentes, dans lequel la structure de renforcement implantable est allongée et l'agencement de détection de déformation est conçu pour mesurer une contrainte sur une plage de sites le long de la structure de renforcement, comportant facultativement une logique de traitement pour déterminer de manière sélective une mesure représentative de contrainte dans la structure de renforcement à proximité d'une site de lésion.

9. Système selon l'une des revendications précédentes, dans lequel l'alerte sonore et/ou visuelle à l'utilisateur comprend au moins l'un d'une instruction textuelle et/ou audio, d'un graphique, d'une animation, d'une lumière, ou d'un son.

10. Système selon l'une des revendications précédentes, dans lequel l'élément musculo-squelettique est un os interne fracturé, dans lequel le dispositif de détection est implantable et encapsulé dans un boîtier ou un revêtement biocompatible, et dans lequel la structure de renforcement est une structure de renforcement osseux entièrement implantée dans le patient, de préférence, dans lequel le dispositif de détection est physiquement couplé à la structure de renforcement à l'aide d'un adhésif biocompatible, de préférence un silicone, et dans lequel l'adhésif biocompatible fournit le boîtier ou le revêtement biocompatible.

11. Système selon l'une des revendications précédentes, dans lequel le dispositif utilisateur est fourni par une application fonctionnant sur un dispositif mobile, facultativement un smartphone, une tablette, un ordinateur ou une montre intelligente.

12. Système selon l'une des revendications précédentes, dans lequel l'agencement d'alimentation électrique est agencé pour recevoir de la puissance d'une source d'alimentation externe, de préférence par induction en utilisant un signal énergétique, facultativement dans lequel l'agencement d'alimentation électrique comprend un condensateur configuré pour se charger par induction en utilisant le signal énergétique, facultativement dans lequel le signal énergétique a une puissance d'environ 500 µW ou moins et est transmis pendant une durée d'environ 2 secondes ou moins, de préférence d'environ 1 seconde, de préférence dans lequel le signal énergétique est un signal radiofréquence, facultativement dans lequel le dispositif utilisateur comprend en outre un appareil de transmission du signal énergétique pour alimenter en puissance le dispositif de détection par induction ou dans lequel le système comprend en outre un dispositif de puissance pour transmettre le signal énergétique pour alimenter en puissance le dispositif de détection par induction, facultativement dans lequel le processeur est conçu pour communiquer avec le dispositif utilisateur via le dispositif de puissance.

13. Système selon l'une des revendications précédentes, dans lequel le processeur est configuré pour mettre le capteur sous tension à des intervalles prédéterminés et créer un journal de lectures dans une mémoire, et dans lequel le processeur est configuré pour transmettre le journal de lectures lors de réception d'une requête provenant du dispositif utilisateur, facultativement dans lequel l'agencement d'alimentation électrique est configuré pour fournir de la puissance au capteur pendant une durée prédéterminée pour chaque mesure, de préférence pas plus de 10 ms, plus préférablement environ 1 ms.

14. Système selon l'une des revendications précédentes, comprenant en outre le serveur distant, dans lequel le serveur distant est configuré pour déterminer une condition de consolidation de la fracture sur la base des données de mesure, facultativement dans lequel, lors de la détermination de la condition de consolidation, le serveur compare les données de mesure avec un ou plusieurs seuils et/ou un ou plusieurs signaux de référence, facultativement dans lequel le serveur comprend un moteur d'apprentissage entraîné à l'aide d'un ensemble de données d'entraînement, le moteur d'apprentissage étant configuré pour recevoir des données d'entrée comportant les données de mesure et pour fournir en tant que sortie la condition de consolidation, de préférence une classification ou une valeur indiquant la progression du traitement, dans lequel facultativement le serveur est configuré pour transmettre la condition de consolidation au dispositif utilisateur, et dans lequel le dispositif d'alerte est conçu, en réponse à la réception de la condition de consolidation, pour mettre à jour l'alerte de sorte qu'elle indique que le patient doit adopter une seconde posture, facultativement dans lequel la première interface de communication est configurée pour envoyer le signal pour déclencher le processeur afin qu'il alimente en puissance le capteur optique une fois que le patient a adopté la seconde posture.

15. Système selon l'une des revendications 7 à 14, lorsqu'elles dépendent de la revendication 6, dans lequel la fibre optique est multimodale et/ou dans lequel une mesure indiquant une température est obtenue, facultativement dans lequel le capteur est capable de mesurer des températures comprises entre environ 25 °C et 50 °C, ou de préférence couvrant une plage de 35 °C à 40 °C.
